Europäisches Patentamt

European Patent Office

Office européen des brevets

(19)

(11) Publication number: **0 353 972 B1**

(12) # EUROPEAN PATENT SPECIFICATION

(45) Date of publication of patent specification: **19.01.94**   (51) Int. Cl.⁵: **A61F  13/02**, C09J 7/00

(21) Application number: **89307753.7**

(22) Date of filing: **31.07.89**

(54) **A novel discontinuous adhesive surface.**

(30) Priority: **01.08.88 US 226502**

(43) Date of publication of application:
**07.02.90 Bulletin  90/06**

(45) Publication of the grant of the patent:
**19.01.94 Bulletin  94/03**

(84) Designated Contracting States:
**AT BE DE FR GB IT NL**

(56) References cited:
**EP-A- 0 170 010**
**EP-A- 0 288 749**
**GB-A- 2 081 101**
**GB-A- 2 119 656**

(73) Proprietor: **THE KENDALL COMPANY**
**15 Hampshire Street**
**Mansfield, Massachusetts 02048(US)**

(72) Inventor: **Takemoto, Shiro George**
**310 Sanderson Avenue**
**Dedham Massachusetts 02026(US)**
Inventor: **Etheredge, Robert Winston, III**
**5 Oakhill Road**
**Natick Massachusetts 01760(US)**

(74) Representative: **Kearney, Kevin David**
**Nicholas et al**
**KILBURN & STRODE**
**30 John Street**
**London, WC1N 2DD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates in general to adhesive tapes and especially medical bandages for application to the skin.

During the past decade, major advances have been made in medical adhesive tapes and the literature is replete with processes and compositions for making such tapes. Yet a tape or bandage in which the adhesive is sufficiently aggressive for good adhesion while not damaging to skin is still elusive.

The prior art medical tapes can be categorized into two main groups: rubber-based or acrylic adhesives on a cloth or other suitable backing material. Rubber-based adhesives enjoyed market dominance until the 60's. Since then acrylic adhesives have become more popular. The primary reason for the popularity of acrylic adhesives has been the so-called "hypoallergenic" factor. Rubber-based adhesive tapes contain components such as pale crepe or synthetic rubber, natural resins, antioxidants, plasticizers, filling and colouring agents, many of which come from plant sources which have the inherent problem of source variation and the potential for introduction of irritant and sensitizing agents.

However, the hypoallergenic factor between rubber-based and acrylic adhesives may be overemphasized since skin sensitivity is primarily correlated to the physical process of wearing a tape or bandage which causes changes in the cohesion of skin cells resulting in injury. The type and severity of skin injury varies with the length of time a bandage is worn. The longer a bandage is worn the more hydrated and thus macerated the outer layer of the stratum corneum, the outermost skin layer, becomes. Under moist conditions, the adhesion between the tape adhesive layer and the outermost skin layers is far greater than the internal strength of the stratum corneum resulting in deep and irregular fractures within the layers of the stratum corneum upon removal of the tape. Accordingly, when a bandage is worn for short intervals of time, such as a few minutes, the upper layers of the stratum corneum do not gain significant fluid from the underlying skin layers resulting in brittle-fracture of the surface of the skin. On the other hand, when a bandage is placed on the skin for longer intervals of time, fluid builds up in the upper layers of the stratum corneum which in turn plasticizes these layers and reduces the elastic modulus and increases the extensibility of skin leading to an increase in the work of fracture and increased stress relaxation of the stratum corneum. The net effect is deeper skin fractures.

Trauma to the skin is lessened when adhesive tapes are constructed to allow normal skin moisture to move through the adhesive and the backing into the environment. Accordingly, the medical tape industry has responded by introducing acrylic adhesives with hydrophilic properties and a high moisture transmission rate spread on a fabric or a nonwoven backing. These tapes advantageously maintain the normal moisture content of the outermost skin layers when intact, thereby causing fracture lines to develop near the surface, in the region of the naturally desquamating layers. Consequently only small amounts of stratum corneum are torn when the tape is removed. However, while these tapes advantageously allow for repeated use of a tape with minimal trauma to the skin, they do have disadvantages.

The major disadvantage of acrylic adhesives with hydrophilic properties, is that they may not adhere well. Thus, while they do not disrupt the integrity of the skin and cause infection, they do fall off, thereby exposing the wound directly to environmental contaminants which can cause infection as well as contact with objects which can be painful or injurious.

In summary, the prior art offers a choice between aggressive adhesive tapes that cause disruption to the skin's integrity and thereby increase the likelihood of infection upon removal, or minimally aggressive adhesive tapes that are hydrophilic and allow for moisture transmission, yet easily fall off and promote injury or infection.

While this dilemma remains unsolved, the prior art has offered incomplete solutions, representative of which is U.S. Patent No. 3,811,438 to Economou. This patent lessens moisture accumulation and thereby increases ventilation by proposing adhesive tapes and bandages comprising a flexible backing with an adhesive portion distributed and adhered thereon in the form of adhesive strips extending the entire width of the backing material, alternately spaced with strips of lesser adhesiveness which strips are generally of a width less than each adjacent layer of adhesive, with each strip having a minimum width of about 0.02 inches (0.508 mm).

While strips of adhesive decrease the surface area in contact with the skin and may thereby decrease the area of injury to the skin upon removal, they minimally lessen the severity of internal injury to the skin because the adhesive strips do run along the entire width of the bandage which upon removal causes a continuous fissure in the interior layers of the skin equal to the width of the bandage. Thus even though the surface area of injury is reduced to the area under the adhesive strips, the severity of injury to the internal skin layers remains the same along the width of the bandage since crack propagation is not prevented along this axis.

Similarly, U.S. Patent No. 2,399,545 of Davis describes a tape wherein the adhesive material is applied in various linear patterns of spaced bands reducing the amount of adhesive material up to 50%. Again, the various linear patterns are continuous strips and therefore cause stratum corneum fractures upon removal.

US-A-4,699,792 shows a bandage in which a carrier web carries adhesive caps or dots (2) and interspersed but separated therefrom caps or dots (3) containing medication.

EP-A-0170010 discloses a bandage having micronised areas of adhesive, and it is stated that the number of possibilities is unlimited but that no particular configuration has been found superior to any of the others.

It is therefore the object of this invention to provide an improved adhesive tape that adheres well to the skin while causing minimal disruption to the integrity of the skin upon removal therefrom.

The disadvantages of the prior art are overcome by having the adhesive applied to the backing or other substrate in the form of tiny individually spaced deposits in particular arrays.

According to the present invention an article of manufacture comprises a backing material carrying spaced small individual adhesive deposits and is characterised in that the adhesive deposits are of varying sizes or of varying distribution, some of the deposits being of one height and the remainder of the deposits being of smaller height, or some of the deposits being spaced closer together at at least one edge of the backing material than in the central portion, or both variations being present.

The invention further extends to an adhesive bandage comprising a vapour- and oxygen-permeable backing material carrying on one surface thereof an absorbent pad for placement as a wound dressing and a medical grade adhesive layer for adhering the said bandage to the skin, being afforded by spaced small adhesive deposits and is characterised in that the adhesive deposits are of varying sizes or of varying distribution, some of the deposits being of one height and the remainder of the deposits being of smaller height, or some of the deposits being spaced closer together at at least one edge of the backing material than in the central portion, or both variations being present.

The said adhesive deposits may be uniformly or substantially uniformly spaced. The said adhesive deposits may be arranged on the said backing material in a geometric pattern. The said adhesive deposits may be substantially spherical.

The said adhesive may be acrylic or rubber-based adhesive. The said adhesive preferably comprises a pressure-sensitive medical grade adhesive. The said adhesive deposits may comprise a drug and/or cosmetic release mechanism. The said adhesive deposits may contain medicinal and/or cosmetic agents. The said adhesive deposits may contain one or more compatible or incompatible medicinal and/or cosmetic agents.

The said backing material may be vapour- and oxygen-permeable.

The deposits are preferably configured so that the area of the surface, the contact surface, of the deposit remote from the surface, the attachment surface, by which the deposits are attached to the backing is much smaller.

Conveniently this can be achieved by making the deposits from substantially spherical particles which on adherence to the backing assume a rounded or generally hemispherical shape. The contact surface is thus theoretically a point but in practice is a rounded area due to compression of the adhesive deposit and conformation of the skin to the rounded surface of the deposit.

Clearly other shapes than spherical or rounded could be effective the aim being to keep the contact surface as small as possible commensurate with good attachment between the deposit and the backing.

The other factor which is critical to achieving good use properties for medical bandages and tapes is the open area of the backing preserved between the adhesive deposits.

The percentage open area (OA) is defined herein as the ratio between the plan area of the backing (AB) minus the sum of the plan areas of the attachment surfaces of the deposits (EAD) to (AB), i.e.

$$OA = (AB - EAD)/AB \ \%.$$

The open area must be such as to ensure that the adhesive particles remain separate and discrete or substantially so. The value of OA is at least 10% e.g. at least 20% but may be higher e.g. as high as 65% or more e.g. up to 80% or 95% or 98%, e.g. 10% to 95% or 20% to 80% or 20% to 65%, e.g. 50 to 60%.

The adhesive products contemplated by this invention enjoy the advantage of non-occlusion, breathability, reduced skin damage upon removal, lowered material cost, improved repositionability, as well as providing a substrate for non-occlusive controlled release of medicaments, if desired.

The invention may be put into practice in various ways and a number of specific embodiments will be described by way of example to illustrate the invention with reference to the accompanying drawings, in which:

Figure 1 shows a diagrammatic plan view of a conventional adhesive bandage;

Figure 2 shows a side elevation view of a preferred embodiment of the invention particularly useful for repositioning;

Figure 3 shows a perspective view of a preferred embodiment of the invention depicting the incorporation of a drug release mechanism;

Figure 4 shows a plan view of still a further embodiment of the invention; and

Figure 5 shows an enlarged perspective view of adhesive small separate discrete deposits after removal from the skin.

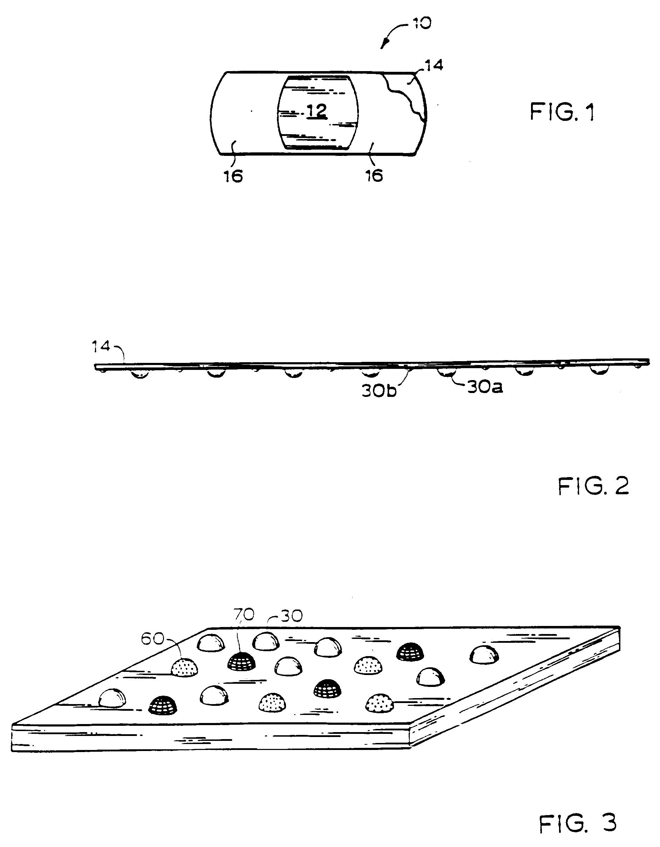

Figure 1 depicts a conventional adhesive bandage 10 comprising an absorbent pad 12 (usually a gauze material), a backing 14, and a continuous adhesive layer 16. As shown, the pad 12 is typically centrally disposed along the length of the adhesive layer to provide sufficient adhesive surface on either side thereof for adhering the bandage to the skin.

Figure 2 illustrates the invention which is designed to improve repositionability. As is used herein and understood in the adhesive art, "repositionability" is defined as the performance of an adhesive relating to its potential for re-adhesion to a substrate after removal. This performance is a function of the amount of unused adhesive available for contact upon a further application of the adhesive to a substrate.

In one preferred embodiment for improved repositionability, as shown in Figure 2, two arrays or patterns of adhesive 16 are printed on the same backing 14, instead of the continuous film of the prior art device shown in Figure 1. One array contains larger size dots 30(a) of greater dimension than the other array of dots 30(b). Upon first application, the dots 30(a) make initial contact with the substrate. Upon repositioning following removal from the substrate, although the adhesive quality of dots 30(a) is diminished, the small dots 30(b) will provide the primary source for adhesion.

Various other design possibilities utilizing a height gradient in the adhesive deposits will be readily suggested to those skilled in the art in view of the foregoing description of Figure 2. Accordingly, it is expressly understood that other design patterns utilizing adhesive deposits of varying sizes and/or shapes to constitute a non-uniform adhesive deposit surface area are contemplated.

Figure 3 depicts the invention's incorporation of a controlled drug or cosmetic release mechanism. By incorporating a topical agent directly into the melted adhesive the same manufacturing process may be utilized for medicament or cosmetic containing adhesive deposits 60, as for adhesive deposits alone 30. The release mechanism may be one of passive diffusion or melting upon contact with the skin. The former process has the advantage of being non-occlusive. This technology may advantageously be extended to the delivery of more than one active ingredient to the skin which is particularly attractive when such ingredients react with one another if housed in the same matrix or when different drug or cosmetic release rates are desired or warranted. In addition, one adhesive deposit 60 may advantageously house the drug or cosmetic while another 70 may house the transport vehicle or a skin conditioner to counter the effect of irritating drugs, or a drug activator.

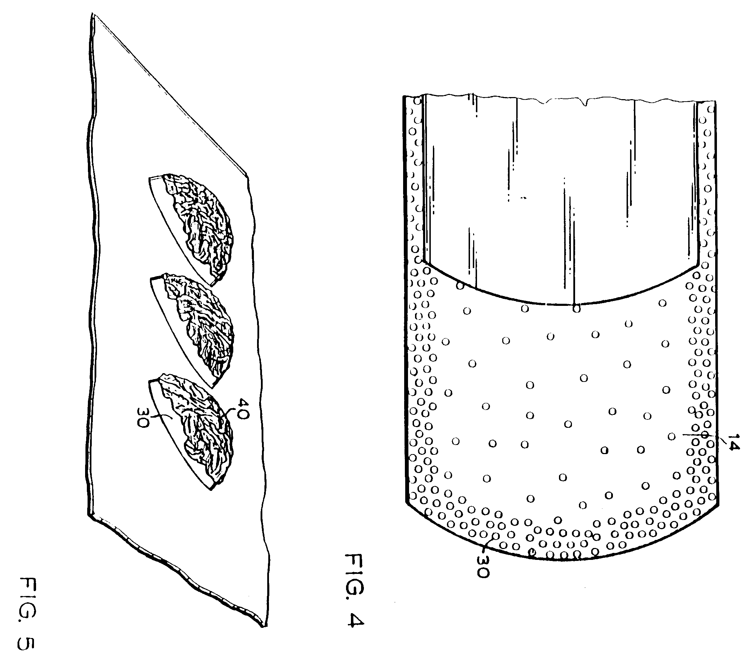

Figure 4 depicts a further embodiment of the invention. Here spherical adhesive deposits 30, are printed in different densities on a given backing. One may for instance apply higher density near the tape edges where shear forces may cause failure of the tape and a lower density as one approaches the central portion to allow skin movement and maximize air and water vapour permeability.

The open area of the pattern at the edges of the bandage is about 80% whilst that in the more open inner region is about 98%.

The backing material 14 may be any of the flexible materials heretofore employed for bandages, e.g. cloth, paper, plastic, strand-reinforced backings or laminates. Preferably, it is either made of a vapour and oxygen permeable material or, e.g. in the case of plastic backings, is perforated to render it permeable to vapour and oxygen. Such backings, which are per se known for bandages, are generally referred to in the art as "breathable".

The adhesive compositions which may be employed are those which preferably are dermatologically acceptable.

By way of illustration, it may be any of the rubber-based or acrylic adhesives of the type generally referred to in the art as being "medical grade" adhesives with a low degree of skin irritation i.e. lower mechanical, chemical and allergic irritation. Preferred are the acrylic adhesives, e.g. comprising a polymer of acrylic acid, an acrylate and an acetic acid ester such as ethyl acetate. However, as previously mentioned, rubber-based adhesive comprising a natural or synthetic rubbery elastomer and a tackifying resin are also contemplated, preferably so long as they are dermatologically acceptable.

The mean particle size or diameter of the individual adhesive deposits to be employed as well as the spacing between individual deposits will in part be dependent upon the aggressiveness of the particular adhesive formulation selected and will in part be dependent upon the desired tack and adhesion properties for the particular type of tape or bandage. The particles may have a diameter or maximum transverse dimension of on the order of from about 20 mils (0.508 mms) to about 10 mils (0.254 mms) may be employed (e.g. 250 to 500 microns). The particles may be deposited so as to provide on the order of from about 200 to about 1000 or about 200 to about 7000, especially about 900 to about 4900, adhesive particles per square inch (30 to 155, 30 to 1100 or 140 to 760 per square cm) of surface area. By way of illustration and as preferred embodiments, however, particles may be deposited by use of screens on the order of 30 to 70 mesh (which have 30 openings by 30 openings per square inch up to 70 openings by 70 openings per square inch). The deposits may provide a weight of adhesive of from about 30 to about 10 grams per square yard (36 to 12 grams per square metre) of surface area.

While the particular particles may be of various shapes, as will be discussed hereinafter in more detail, generally spherical particles which assume a hemispherical or rounded shape on the backing are most preferred.

The attachment of the adhesive deposits 30 to the backing 14 may be made by activating the adhesive in any of the known ways such as by solvent, heat or pressure. The preferred embodiment of the invention comprises a hot melt pressure sensitive adhesive, as it allows for the highest degree of precision in the placement of the adhesive. Among the hot melt application equipment that may be used, mention may be made of slot orifice coaters, roll coaters, extrusion coaters, screen process printing and gravure coating. The latter two are the preferred method for applying the inventive adhesive pattern due to their precision. Both gravure and screen printing are well known and per se comprise no part of this invention. Accordingly, they need not be described in great detail.

Test results have illustrated that hot melt pressure sensitive adhesive applied in a discontinuous pattern of dots, has numerous advantages among them non-occlusion and breathability, reduced skin damage, lower manufacturing cost, improved repositionability as well as being an excellent substrate for non-occlusive controlled release of medicaments as illustrated in Figure 4. Medical bandages of the present invention include wound dressings, finger bandages, blister and contusion prevention bandages and athletic tape. Industrial tapes in accordance with the present invention include reinforcement tape, harness tape, duct tape and masking tape.

The following sets forth two examples of manufacture of a medical tape and a control and the corresponding test results.

## EXAMPLE I

Microsize Backing - non woven sized with acrylic polymer. Rubber-based Hot Melt Pressure Sensitive Adhesive hereafter (HMPSA).
Hot screen printed on a Kraemer Coating apparatus with a 60 mesh screen (which has 60 openings by 60 openings per square inch). Dot pattern at 11 gram/yd$^2$ (13 gram/m$^2$).
Adhesive dots approximately .012" (0.30 mm) diameter.
Adhesive dot distribution 3600/in$^2$ (558/cm$^2$).
Open area: 59%
Adhesion to Steel 17.7 oz/in (197.5 gram/cm).
Tack 150 g.
Porosity
Microsize without adhesive, 0.180 sec/100cc/in$^2$ (0.028 sec/100cc/cm$^2$).
Microsize with adhesive dots, 0.197 sec/100cc/in$^2$ (0.031 sec/100cc/cm$^2$).

## EXAMPLE II

Microsize Backing - non woven sized with acrylic polymer.Rubber-based HMPSA Adhesive.
Hot screen printed on a Kraemer Coating apparatus with a 50 Mesh Screen (which has 50 openings by 50 openings per square inch). Dot pattern at 15 gram/yd$^2$ (18 grams/m$^2$).
Adhesive dots approximately 0.015" (0.38 mm) diameter.
Adhesive dot distribution about 2500/in$^2$ (388/cm$^2$).
Open area: 56%
Adhesion to Steel: 56 oz/in (625 gram/cm).
Tack: 140 g.
Porosity
Microsize: without adhesive, 0.181 sec/100cc/in$^2$ (0.028 sec/100cc/cm$^2$).
Microsize: with adhesive dots, 0.253sec/100cc/in$^2$ (0.039 sec/100cc/cm$^2$).

## EXAMPLE III (Control)

Microsize Backing.
Rubber based HMPSA.
Adhesion to Steel: 70.3 oz/in (785 gram/cm).
Tack: 591 g
Porosity
Microsized without adhesive, 0.181 sec/100cc/in$^2$ (0.028 sec/100cc/cm$^2$).
Microsized with continuous adhesive film 9.25 sec/100cc/in$^2$ (1.43 sec/100cc/cm$^2$).

These examples demonstrate a positive correlation between mesh size and hence adhesive dot diameter, and porosity. Since an inverse relationship exists between tape porosity and skin maceration, one may deduce that the greater the degree of porosity the less skin damage will occur. The examples of manufacture illustrate a marked increase in porosity of 0.197 sec/100cc/in$^2$ (0.031 sec/100cc/cm$^2$) and 0.253 sec/100cc/in$^2$ (0.039 sec/100cc/cm$^2$) as opposed to 9.25 sec/100cc/in$^2$ (1.43 sec/100cc/cm$^2$) of a solid rubber based adhesive. Simply stated it takes 0.197-0.253 seconds for 100 cc of air to be forced through a discontinuous adhesive film, tape or bandage, as opposed to 9.25 seconds for a continuous adhesive film, tape or bandage. Thus the invention reduces exposure of skin to moisture and consequently reduces skin maceration and skin damage upon removal of the tape or bandage.

The examples further illustrate a marked decrease in adhesion, 17.7 oz/in (197.5 gram/cm) and 56 oz/in (625 gram/cm) as opposed to 70.3 oz/in (785 gram/cm) of a continuous rubber based adhesive film, tape or bandage. Notably this decrease is due to the decreased amount of adhesive used in the discontinuous as opposed to a continuous adhesive surface as the type of adhesive remained constant. However this decrease in adhesion is offset by the invention's ability to incorporate a much more aggressive adhesive.

A continuous adhesive surface is restricted to less aggressive adhesives to minimize skin damage upon removal whereas a discontinuous adhesive surface by nature of reduced skin/adhesive contact may employ a much more aggressive adhesive. Thus even though the amount of adhesive used is decreased, the adhesive strength need not be decreased. In sum, the invention advantageously allows one to use a smaller amount of adhesive yet a more aggressive type of adhesive so as to be competitive with the adhesive strength of a prior art continuous adhesive film, tape or bandage. Naturally aside from the usual effect of temperature, moisture, and oil presence on the surface to be bonded, hot screen printed HMPSA performance on skin will depend on:

1. Deposit Size (controlled by mesh size and melt viscosity).
2. Deposit Distribution (screen design).
3. Type of HMPSA (softness, cohesive strength, aggressiveness).
4. Type of Backing - Smooth backing requires less adhesive than very rough backing.
5. Anchorage of adhesive to backing.

Thus, each specific backing will require its own adhesive design and/or HMPSA for it to function satisfactorily.

## Claims

1. An adhesive tape or an adhesive bandage comprising a backing material (14) carrying spaced small individual adhesive deposits (30, 30a, 30b) on a surface thereof, characterised in that the adhesive deposits are of varying sizes or of varying distribution, some of the deposits being of one height (30a) and the remainder of the deposits being of smaller height (30b), or some of the deposits (30) being spaced closer together at at least one edge of the backing material (14) than in the central portion, or both variations being present.

2. An adhesive tape or an adhesive bandage comprising a vapour- and oxygen-permeable backing material (14) carrying on one surface thereof an absorbent pad (12) for placement as a wound dressing and a medical grade adhesive layer for adhering said bandage to the skin, being afforded by spaced small individual adhesive deposits (30, 30a, 30b), characterised in that the adhesive deposits are of varying sizes or of varying distribution, some of the deposits being of one height (30a) and the remainder of the deposits being of smaller height (30b), or some of the deposits (30) being spaced closer together at at least one edge of the backing material (14) than in the central portion, or both variations being present.

3. An article as claimed in Claim 1 or Claim 2 characterised in that the said adhesive deposits (30a, 30b) are of varying size.

4. An article as claimed in any one of Claims 1 to 3 characterised in that the said adhesive deposits (30) are more densely positioned along at least one edge than in the central portion of the said backing material.

5. An article as claimed in any one of Claims 1 to 4 characterised in that some of the said adhesive deposits contain a first material (60) and others of the adhesive deposits (70) contain other materials which may or may not be compatible with each other or with the first material, incompatible materials being located separate from each other in different deposits.

6. An article as claimed in any one of Claims 1 to 4 characterised in that the said adhesive deposits (60, 70) contain one or more compatible or incompatible medicinal and/or cosmetic agents.

7. An article as claimed in any one of claims 1 to 4 characterised in that certain of the adhesive deposits (60) contain a drug or cosmetic active ingredient and others of the deposits (70) contain a transport vehicle or a skin conditioner or a drug activator and yet others of the deposits (30) contain none of such ingredients.

8. An article as claimed in any one of Claims 1 to 7 in which there are 30 to 1100 deposits of adhesive per $cm^2$.

9. An article as claimed in any one of Claims 1 to 8 in which the deposits leave 10% to 95% open area which is adhesiveless on the backing sheet.

**Patentansprüche**

1. Klebeband oder Klebebandage, bestehend aus einem Unterlagsmaterial (14), das an einer seiner Seiten kleine, im Abstand angeordnete, einzelne Klebstoffbelegungen (30, 30a, 30b) trägt, dadurch gekennzeichnet, daß die Klebstoffbelegungen unterschiedlich groß oder unterschiedlich verteilt sind, wobei einige der Belegungen eine Höhe (30a) aufweisen und die übrigen Belegungen eine geringere Höhe (30b) aufweisen oder wobei einige der Belegungen (30) an zumindest einem Rand des Unterlagsmaterials (14) in kleinerem gegenseitigem Abstand als im Mittelbereich angeordnet sind oder beide Varianten vorhanden sind.

2. Klebeband oder Klebebandage, bestehend aus einem dampf- oder sauerstoffdurchlässigen Unterlagsmaterial (14), das an einer seiner Seiten ein absorbierendes Kissen (12) zum Auflegen als Wundverband und eine medizinische Klebeschicht zum Aufkleben der Bandage auf die Haut trägt, wobei dies durch kleine, im Abstand angeordnete, einzelne Klebstoffbelegungen (30, 30a, 30b) erzielt wird, dadurch gekennzeichnet, daß die Klebstoffbelegungen unterschiedlich groß oder unterschiedlich verteilt sind, wobei einige der Belegungen eine Höhe (30a) aufweisen und die übrigen Belegungen eine geringere Höhe (30b) aufweisen oder wobei einige der Belegungen (30) an zumindest einem Rand des Unterlagsmaterials (14) in kleinerem gegenseitigem Abstand als im Mittelbereich angeordnet sind oder beide Varianten vorhanden sind.

3. Gegenstand nach Anspruch 1 oder 2, dadurch gekennzeichnet, daß die Klebstoffbelegungen (30a, 30b) unterschiedlich groß sind.

4. Gegenstand nach den Ansprüchen 1 bis 3, dadurch gekennzeichnet, daß die Klebstoffbelegungen (30) längs zumindest eines Randes dichter angeordnet sind als im Mittelbereich des Unterlagsmaterials.

5. Gegenstand nach den Ansprüchen 1 bis 4, dadurch gekennzeichnet, daß einige der Klebstoffbelegungen ein erstes Material (6) enthalten und andere der Klebstoffbelegungen (70) andere Materialien enthalten, die miteinander oder mit dem ersten Material kompatibel sein können oder nicht, wobei inkompatible Materialien voneinander getrennt in verschiedenen Belegungen angeordnet sind.

6. Gegenstand nach den Ansprüchen 1 bis 4, dadurch gekennzeichnet, daß die Klebstoffbelegungen (60, 70) einen oder mehrere kompatible oder inkompatible medizinische und/oder kosmetische Wirkstoffe enthalten.

7. Gegenstand nach den Ansprüchen 1 bis 4, dadurch gekennzeichnet, daß gewisse der Klebstoffbelegungen (60) einen medikamentösen oder kosmetisch aktiven Bestandteil und andere der Belegungen (70) ein Transportmittel oder ein Hautbehandlungsmittel oder einen Medikamentenaktivator und weitere andere Belegungen (30) keine solchen Bestandteile enthalten.

8. Gegenstand nach den Ansprüchen 1 bis 7, dadurch gekennzeichnet, daß es 30 bis 1100 Klebstoffbelegungen pro cm$^2$ gibt.

9. Gegenstand nach den Ansprüchen 1 bis 8, dadurch gekennzeichnet, daß die Belegungen einen offenen Bereich von 10% bis 95% freilassen, der auf der Unterlagsbahn klebstofffrei ist.

**Revendications**

1. Ruban adhésif ou bandage adhésif comprenant un matériau de support (14) portant de petits dépôts d'adhésif, individuels et espacés (30, 30a, 30b) sur l'une de ses surfaces, qui se caractérise en ce que les dépôts d'adhésif possèdent des dimensions variables ou sont distribués de manière variable, certains des dépôts sont d'une hauteur (30a) et le reste des dépôts sont d'une hauteur plus faible (30b), ou bien certain des dépôts (30) sont plus rapprochés les uns des autres à au moins un bord du matériau de support (14) que dans la partie centrale de celui-ci, ou bien les deux variétés sont présentes.

2. Ruban adhésif ou bandage adhésif, comprenant un matériau de support (14) perméable à la vapeur et à l'oxygène portant, sur l'une de ses surfaces, une compresse absorbante (12) pour le placement en tant que pansement sur une plaie et une couche adhésive de qualité médicale pour faire adhérer le bandage à la peau, qui est pourvue de petits dépôts d'adhésif, individuels et espacés (30, 30a, 30b) sur l'une de ses surfaces, qui se caractérise en ce que les dépôts d'adhésif possèdent des dimensions variables ou sont distribués de manière variable, certains des dépôts sont d'une hauteur (30a) et le reste des dépôts sont d'une hauteur plus faible (30b), ou bien certain des dépôts (30) sont plus rapprochés les uns des autres à au moins un bord du matériau de support (14) que dans la partie centrale de celui-ci, ou bien les deux variétés sont présentes.

3. Article suivant la revendication 1 ou la revendication 2, caractérisé en ce que les dépôts d'adhésif (30a, 30b) sont de dimensions variables.

4. Article suivant l'une quelconque des revendications 1 à 3, caractérisé en ce que les dépôts d'adhésif (30) sont disposés de manière plus dense le long d'au moins un bord que dans la partie centrale du matériau de support précité.

5. Article suivant l'une quelconque des revendications 1 à 4, caractérisé en ce que certains des dépôts d'adhésif contiennent un premier matériau (60) et d'autres parmi ces dépôts d'adhésif (70) contiennent d'autres matériaux qui peuvent être ou ne pas être compatibles les uns avec les autres ou avec le premier matériau, les matériaux incompatibles étant logés séparément les uns des autres en dépôts différents.

6. Article suivant l'une quelconque des revendications 1 à 4, caractérisé en ce que les dépôts d'adhésif (60, 70) contiennent un ou plusieurs agents médicinaux et/ou cosmétiques, compatibles ou incompatibles.

7. Article suivant l'une quelconque des revendications 1 à 4, caractérisé en ce que certains des dépôts d'adhésif (60) contiennent un médicament ou un ingrédient cosmétique actif, cependant que d'autres de ces dépôts (70) contiennent un véhicule de transport ou un agent de conditionnement de la peau, ou un activateur de médicament, alors que d'autres encore de ces dépôts (30) ne contiennent aucun de tels ingrédients.

8. Article suivant l'une quelconque des revendications 1 à 7, caractérisé en ce qu'il existe de 30 à 1100 dépôts d'adhésif par cm².

9. Article suivant l'une quelconque des revendications 1 à 8, caractérisé en ce que les dépôts laissent subsister de 10% à 95% de surface ouverte, qui n'adhèrent pas à la feuille de support.

FIG. 1

FIG. 2

FIG. 3

FIG. 4

FIG. 5

10